(19) Europäisches Patentamt
European Patent Office
Office européen des brevets

(11) EP 1 348 421 A2

(12) DEMANDE DE BREVET EUROPEEN

(43) Date de publication:
**01.10.2003 Bulletin 2003/40**

(51) Int Cl.[7]: **A61K 7/11**, A61K 7/06

(21) Numéro de dépôt: **03290598.6**

(22) Date de dépôt: **11.03.2003**

(84) Etats contractants désignés:
**AT BE BG CH CY CZ DE DK EE ES FI FR GB GR HU IE IT LI LU MC NL PT RO SE SI SK TR**
Etats d'extension désignés:
**AL LT LV MK**

(30) Priorité: **25.03.2002 FR 0203710**
**28.11.2002 FR 0214973**

(71) Demandeur: **L'OREAL**
**75008 Paris (FR)**

(72) Inventeurs:
- **Quinn, Francis Xavier**
**75005 Paris (FR)**
- **Pruche, Francis**
**60300 Senlis (FR)**
- **Mahe, Yann**
**91390 Morsang sur Orge (FR)**

(74) Mandataire: **Bourdeau, Françoise**
**L'OREAL,**
**Département Propriété Industrielle,**
**6, rue Bertrand Sincholle**
**92585 Clichy Cedex (FR)**

# (54) Procédé de densification ou de coiffage des fibres kératiniques humaines utilisant un système catalytique particulier

(57) L'invention se rapporte à un procédé de densification des cheveux, comprenant une étape d'application sur les cheveux et/ou le cuir chevelu d'une composition comprenant, dans un milieu physiologiquement acceptable, une quantité efficace d'au moins un précurseur de densification choisi parmi les composés comportant au moins un cycle aromatique ayant au moins deux groupes hydroxyles portés par deux atomes de carbone consécutifs du cycle aromatique et une quantité efficace d'un système catalytique comprenant un premier constituant choisi parmi les sels et oxydes de Mn(II) et/ou de Zn(II) et leurs mélanges et un second constituant choisi parmi les hydrogénocarbonates alcalins, les hydrogénocarbonates alcalino-terreux et leurs mélanges.

Elle se rapporte aussi à une composition contenant dans un milieu physiologiquement acceptable lesdits précurseur de densification et système catalytique, associés à un actif favorisant la repousse des cheveux, à effet retard ou pour le coiffage des cheveux.

EP 1 348 421 A2

## Description

La présente invention se rapporte à un procédé de densification immédiate des zones alopéciques et à l'utilisation de l'association d'un précurseur de densification et d'un système catalytique doux comme agent de densification des fibres kératiniques et en particulier des cheveux d'êtres humains. Elle se rapporte aussi à une composition cosmétique ou dermatologique contenant cette association précurseur-système catalytique en présence d'un actif favorisant la repousse des fibres kératiniques et/ou limitant leurs chutes, à effet retard ou pour le coiffage des cheveux.

La croissance des fibres kératiniques et plus spécialement des cheveux et leur renouvellement sont principalement déterminés par l'activité des follicules pileux et de leur environnement matriciel. Leur activité est cyclique et comporte essentiellement trois phases à savoir la phase anagène, la phase catagène et la phase télogène.

A la phase anagène (phase active ou de croissance), qui dure plusieurs années et au cours de laquelle les cheveux s'allongent, succède une phase catagène très courte et transitoire qui dure quelques semaines. Au cours de cette phase, le cheveu subit une évolution, le follicule s'atrophie et son implantation dermique apparaît de plus en plus haute.

La phase terminale ou phase télogène, qui dure quelques mois, correspond à une phase de repos du follicule et le cheveu finit par tomber. A la fin de cette période de repos, un nouveau follicule est régénéré, sur place, et un autre cycle recommence.

La phase terminale ou phase télogène, qui dure quelques mois, correspond à une phase de repos du follicule et le cheveu finit par tomber. A la fin de cette période de repos, un nouveau follicule est régénéré, sur place, et un autre cycle recommence.

La chevelure se renouvelle donc en permanence et sur les 150 000 cheveux environ que comporte une chevelure, 10% environ sont au repos et seront remplacés en quelques mois.

La chute ou perte naturelle des cheveux peut être estimée, en moyenne, à quelques cent cheveux par jour pour un état physiologique normal. Ce processus de renouvellement physique permanent subit une évolution naturelle au cours du vieillissement, les cheveux deviennent plus fins et leurs cycles plus courts.

En outre, différentes causes peuvent entraîner une perte importante, temporaire ou définitive, des cheveux. Il peut s'agir de chute et d'altération des cheveux au décours d'une grossesse (post partum), au cours d'états de dénutrition ou de déséquilibres alimentaires ou encore au cours d'états d'asthénie ou de dysfonctionnement hormonal comme cela peut être le cas au cours ou au décours de la ménopause. Il peut également s'agir de chute ou d'altérations des cheveux en relation avec des phénomènes saisonniers.

Il peut s'agir également d'une alopécie, qui est essentiellement due à une perturbation du renouvellement capillaire entraînant dans un premier temps l'accélération de la fréquence des cycles au détriment de la qualité des cheveux, puis de leur quantité. Les cycles de croissance successifs aboutissent à des cheveux de plus en plus fins et de plus en plus courts, se transformant peu à peu en un duvet non pigmenté. Des zones sont touchées préférentiellement, notamment les golfes temporaux ou frontaux chez l'homme et, chez la femme, on constate une alopécie diffuse du vertex.

Le terme alopécie recouvre aussi toute une famille d'atteintes du follicule pileux ayant pour conséquence finale la perte définitive, partielle ou générale des cheveux. Il s'agit plus particulièrement de l'alopécie androgénique. Dans un nombre important de cas, la chute précoce des cheveux survient chez des sujets prédisposés génétiquement, il s'agit alors d'alopécie andro-chrono-génétique ; cette forme d'alopécie concerne notamment les hommes.

Il est connu, par ailleurs, que certains facteurs tels qu'un déséquilibre hormonal, un stress physiologique, la malnutrition, peuvent accentuer le phénomène.

Dans certaines dermatoses du cuir chevelu à caractéristique inflammatoire, telles que par exemple le psoriasis ou les dermatites séborrhéïques, la chute des cheveux peut être fortement accentuée ou entraîner des cycles des follicules fortement perturbés.

On recherche depuis de nombreuses années, dans l'industrie cosmétique ou pharmaceutique, des compositions permettant de supprimer ou de réduire l'alopécie, et notamment d'induire ou de stimuler la croissance des cheveux ou de diminuer leur chute ou pour le coiffage des cheveux.

Les compositions actuellement utilisées présentent l'inconvénient de ne faire apparaître la densification des cheveux que plusieurs jours, voire plusieurs mois, après le traitement, ce qui pour certaines personnes peut être très gênant voir endicapant. Ainsi, pour le finastéride, il faut attendre de 9 à 12 mois pour percevoir un bénéfice, pour le minoxidil ou l'aminexil, l'utilisateur commence à avoir un bénéfice après 1, 5 mois et plus généralement au bout de 3 à 6 mois. Or, à ce jour, il n'existe pas de compositions à densification capillaire immédiate.

Par ailleurs, les actifs à action longue sont en général appliqués de façon quotidienne, voire parfois bi-quotidienne, ce qui suscite une très grande attente de la part de l'utilisateur qui parfois, n'observant pas d'effet après l'utilisation de son premier flacon ou de sa première prescription ne renouvelle pas l'application au delà. On dit alors que l' «observance » (c'est-à-dire le suivi des recommandations d'utilisation en termes de dose et de durée) n'est pas bonne.

Par ailleurs entrer dans une stratégie antichute nécessite qu'un seuil de tolérance de son état alopécique soit dépassé. L'utilisateur a donc besoin d'un effet immédiatement visible et perceptible. Ceci est de plus particulièrement adapté aux demandes actuelles des utilisateurs en termes de facilité et rapidité d'exécution du traitement.

Les produits capillaires pour la fixation des cheveux les plus répandus sur le marché de la cosmétique sont des compositions à pulvériser en aérosol ou en flacon pompe tels que les laques, les sprays ou les mousses, essentiellement constituées d'une solution le plus souvent alcoolique ou hydroalcoolique et d'un polymère filmogène soluble dans l'eau ou dans l'alcool, en mélange avec divers adjuvants cosmétiques.

De nombreuses compositions de maintien et/ou de mise en forme des cheveux de l'art antérieur, qui contiennent dans leur formulation des polymères de coiffage, peuvent altérer les propriétés cosmétiques des cheveux. Ceux-ci peuvent, après application de telles compositions, devenir rêches, perdre leur douceur naturelle ou leur aspect agréable.

On recherche donc des polymères pour les compositions capillaires, notamment pour le maintien et/ou la fixation de la coiffure, qui procurent à la chevelure, outre un maintien et/ou une fixation satisfaisante, de bonnes propriétés cosmétiques, en particulier un bon démêlage, de la douceur et un toucher agréable.

Néanmoins, l'utilisation de ces polymères présente plusieurs inconvénients. Le premier réside dans le fait que, lorsque les polymères sont utilisés au-delà d'une certaine concentration, les compositions s'appliquent difficilement (la viscosité augmente). Cela crée des surcharges à certains endroits de la chevelure et donc des défauts cosmétiques et cela implique aussi que certaines parties de la chevelure reçoivent moins de composition, ce qui, au final, induit sur ces parties, un effet moindre.

Le second inconvénient réside dans le fait qu'ils sont parfois difficiles à mettre en oeuvre. En effet, les composés polymériques à faible solubilité dans l'eau exigent l'utilisation de solvant organique ou mélange de solvants organiques. Cela crée plusieurs problèmes induits, notamment problème d'environnement, effet sur la cosméticité des cheveux. Certains composés polymériques peuvent être utilisés dans l'eau sans ajout d'un quelconque co-solvant. Dans ces cas, la limitation réside dans le fait que ces composés polymériques sont éliminés partiellement, voire totalement, par rinçage des cheveux. Ainsi, l'effet dû au composé polymérique est très limité après rinçage. Finalement, cela limite l'effet des traitements rincés (shampooing, après-shampooing), et réduit aussi l'intérêt des compositions utilisées en mode non rincé (laques, mousses, lotions de mise en plis ...), dans la mesure où l'utilisateur perd l'effet acquis par le traitement lorsqu'il se lave les cheveux.

Il est possible d'obtenir un effet cosmétique efficace, résistant au rinçage, sans risquer les problèmes de cheveux chargés en cas de superposition et sans être obligés d'utiliser des solvants organiques. Pour cela, on utilise des composés organosiloxanes sous forme de monomères solubles dans l'eau présentant au moins une fonction chimique neutralisable, qu'on introduit dans l'eau, et auxquels on ajoute un agent régulateur de pH dans des proportions telles que les composés organosiloxanes se trouvent en partie mais non totalement neutralisés.

Dans ces cas, on observe que l'application de ces compositions permettent d'obtenir des effets cosmétiques marqués, sans problème en cas de superposition. Ces effets résistent au rinçage. De telles compositions sont décrites dans les brevets FR 2.783.164, FR 2.783.167 et FR 2.783.165.

Malgré les effets intéressants observés, cette méthode présente un inconvénient majeur: il s'agit du pH du produit. Afin d'obtenir les effets intéressants, le pH de la formule doit être supérieur à 9, voire 10. Il est connu à l'homme de l'art que l'application répétée sur les cheveux de produits avec un pH élevé peut induire un affaiblissement des cheveux.

De manière surprenante et inattendue, la Demanderesse a découvert qu'il était possible de répondre aux problèmes ci-dessus en utilisant un mélange d'un othodiphénol et d'un système catalytique comprenant un sel et/ou oxyde de Mn(II) et/ou de Zn(II) et un hydrogénocarbonate d'un métal alcalin.

Dans la présente demande, on entend par effet coiffant le maintien et/ou la fixation de la coiffure, ce maintien et/ou cette fixation de la coiffure étant de préférence s'accompagnant entre autres d'une ou plusieurs des propriétés suivantes : douceur, toucher agréable, bon démêlage, lissage, volume et gonflant.

L'invention a justement pour objet un procédé de densification immédiate des fibres kératiniques humaines et plus spécialement des cheveux permettant notamment de compenser rapidement la perte de cheveux et d'être couplé à un procédé de densification à temps de réponse plus long tel que ceux utilisé jusqu'à ce jour ou pour le coiffage des cheveux.

De façon plus précise l'invention a pour objet un procédé de densification des fibres kératiniques humaines comprenant une étape qui consiste à appliquer sur les fibres kératiniques et la peau d'où émergent les dites fibres et plus spécialement sur les cheveux et/ou le cuir chevelu, une composition cosmétique contenant, dans un milieu physiologiquement acceptable à application topique, une quantité efficace d'au moins un précurseur de densification, choisi parmi les composés comportant au moins un cycle aromatique ayant au moins deux groupes hydroxyles portés par deux atomes de carbone consécutifs du cycle aromatique, et une quantité efficace d'un système catalytique comprenant un premier constituant choisi parmi les sels et oxydes de Mn(II) et/ou de Zn(II) et leurs mélanges et un second constituant choisi parmi les hydrogénocarbonates alcalins, les hydrogénocarbonates alcalino-terreux et leurs mélanges, les proportions du premier et du second constituants étant telles que:

$$\frac{[Mn(II)]}{[HCO_3]} \leq 1 \text{ avec } [Mn(II)] \neq 0$$

$$\frac{[Zn(II)]}{[HC0_3]} \leq 1 \text{ avec } [Zn(II)] \neq 0$$

$$\frac{[Mn(II) + Zn(II)]}{[HCO_3]} \leq 1 \text{ avec } [Mn(II)] \text{ et } [Zn(II)] \neq 0$$

Ce procédé peut comprendre, en outre, une étape de contact avec les fibres kératiniques et/ou de la peau d'où émergent les dites fibres, et en particulier des cheveux et/ou du cuir chevelu, du précurseur de densification et du système catalytique et éventuellement une étape de rinçage des fibres et peau adjacente aux dites fibres.

Ce procédé de traitement présente les caractéristiques d'un procédé cosmétique dans la mesure où il permet d'améliorer l'esthétique des cheveux en leur donnant une plus grande vigueur et un aspect amélioré. En outre, il peut être utilisé quotidiennement pendant plusieurs mois.

Ce procédé présente l'avantage d'être rapide à mettre en oeuvre, et de nécessiter un temps de pose de 1 à 30 min, par exemple de 3 à 15 min. Au bout de ce temps de pose, la densité capillaire apparaît comme fortement accrue.

L'invention se rapporte encore à l'utilisation de l'association d'au moins un précurseur de densification et d'un système catalytique tels que définis précédemment, comme agent pour augmenter la densification des fibres kératiniques, et notamment des cheveux et/ou augmenter leur densité.

L'invention se rapporte encore à l'utilisation cosmétique d'au moins un précurseur de densification et d'un système catalytique, tels que définis précédemment, dans une composition cosmétique de soin capillaire d'être humain, pour augmenter la densité des fibres kératiniques humaines et notamment des cheveux. Elle a encore pour objet l'utilisation d'au moins un précurseur de densification et d'un système catalytique, tels que définis précédemment, pour la préparation d'une composition capillaire pour être humain, destinée à augmenter la densité des fibres kératiniques humaines telles que les cheveux et les cils ou pour le coiffage des cheveux.

En particulier, l'invention se rapporte à l'utilisation cosmétique de l'association d'au moins un précurseur de densification et d'un système catalytique, tels que définis précédemment, dans une composition cosmétique de soin capillaire d'être humain pour traiter l'alopécie androgénique ou encore à l'utilisation de l'association d'au moins un précurseur de densification et d'un système catalytique, tels que définis précédemment, pour la préparation d'une composition capillaire d'être humain destinée à traiter l'alopécie androgénique. Ainsi, cette composition permet de maintenir en bon état la chevelure et/ou compenser de façon immédiate la chute naturelle des cheveux des hommes ou elle pertmet le coiffage des cheveux.

Le système catalytique chimique présent dans la composition utilisable dans l'invention se comporte comme une pseudo-oxydase capable de mimer l'activité oxydase sans les inconvénients liés à l'emploi d'un système enzymatique. En particulier, cette composition de densification des fibres kératiniques ne nécessite pas la présence d'enzyme.

La quantité efficace de précurseur de densification et de système catalytique correspond à la quantité nécessaire pour obtenir le résultat désiré (à savoir augmenter la densité des cheveux). L'homme du métier est donc en mesure d'évaluer cette quantité efficace qui dépend de la nature du précurseur utilisé, de la nature du système catalytique utilisé, de la personne à laquelle on applique le précurseur de densification et le système catalytique ainsi que du temps de cette application.

Dans la suite du texte, et sauf indication contraire, les quantités des différents ingrédients de la composition sont données en pourcentage en poids par rapport au poids total de la composition.

La composition utilisable dans l'invention comprend, donc, dans un milieu physiologiquement acceptable, a) une quantité efficace d'au moins un précurseur de densification choisi parmi les composés comportant au moins un cycle aromatique ayant au moins deux groupes hydroxyles (OH) portés par deux atomes de carbone consécutifs du cycle aromatique et b) une quantité efficace d'un système catalytique comprenant un premier constituant choisi parmi les sels et les oxydes de Mn(II), les sels et les oxydes de Zn(II), les sels et les oxydes doubles de Mn(II) et de Zn(II) et les mélanges de ces oxydes et de ces sels, et un second constituant choisi parmi les hydrogénocarbonates alcalins, les hydrogénocarbonates alcalino-terreux et leurs mélanges, les proportions du premier constituant et du second constituants étant telles que :

$$\frac{[Mn(II)]}{[HCO_3]} \leq 1 \text{ avec } [Mn(II)] \neq 0$$

$$[\frac{Zn(II)]}{[HC0_3]} \leq 1 \text{ avec } [Zn(II)] \neq 0$$

$$\frac{[Mn(II) + Zn(II)]}{[HCO_3]} \leq 1 \text{ avec } [Mn(II)] \text{ et } [Zn(II)] \neq 0$$

où [Mn(II)], [Zn(II)] et [$HCO_3$] représentent respectivement les concentrations molaires en Mn(II), Zn(II) et $HCO_3$ dans la composition.

Selon l'invention, on peut utiliser un ou plusieurs précurseurs de densification associé(s) à un ou plusieurs sels et/ou oxydes de Mn(II), ou à un ou plusieurs sels et/ou oxydes de Zn(II), ou à un mélange de sels et/ou oxydes de Mn(II) et de sels ou oxydes de Zn(II), voire à un mélange de sels et/ou d'oxydes doubles de Mn(II) et Zn(II).

Généralement, le rapport $\frac{[Mn(II)]}{[HCO_3]}$ est choisi dans la gamme allant de $10^{-5}$ à $10^{-1}$, de préférence de $10^{-3}$ à $10^{-2}$ et est typiquement de l'ordre de $5.10^{-3}$.

Dans le cas où on utilise un sel ou un oxyde de Zn(II) seul, le rapport $\frac{[Zn(II)]}{[HCO_3]}$ est en général d'un ordre de 10 à 100 fois supérieur au rapport dans le cas de l'emploi d'un sel ou oxyde de Mn(II) seul.

Typiquement, ce rapport est de $10^{-4}$ ou plus, de préférence $10^{-3}$ ou plus, et de préférence de l'ordre de $5.10^{-1}$.

Dans le cas d'un mélange de Mn(II) et Zn(II), le rapport varie généralement de $10^{-5}$ à $10^{-1}$, de préférence $10^{-3}$ à $10^{-2}$, ce rapport étant choisi plus élevé lorsque la proportion de Zn(II) dans le mélange s'accroît.

Généralement, la concentration molaire en Mn(II), Zn(II) ou Mn(II)+Zn(II) dans la composition finale varie de $10^{-3}$ mM/l à 10 mM/l, de préférence de $10^{-2}$ mM/l à 1 mM/l.

Lorsqu'on utilise seulement un ou plusieurs sels ou oxydes de Mn(II), la concentration molaire en Mn(II) dans la composition finale est typiquement de $10^{-3}$ mM/l à $10^{-1}$ mM/l, et de préférence de $10^{-2}$ mM/l à $10^{-1}$ mM/l.

En pratique, lorsque le système catalytique comporte seulement un ou plusieurs sels ou oxydes de Zn(II), la concentration en Zn(II) dans la composition finale est de $5.10^{-2}$ mM/l à 10 mM/l, mieux de $5.10^{-1}$ mM/l à 1 mM/l.

Parmi les sels de Mn(II) et Zn(II) convenant pour la présente invention, on peut citer les chlorure, fluorure, iodure, sulfate, phosphate, nitrate et perchlorate, les sels d'acides carboxyliques et leurs mélanges.

A titre d'exemple, on peut citer le chlorure de manganèse, le carbonate de manganèse (par exemple rhodochrosite), le difluorure de Mn(II), l'acétate de Mn(II) tétrahydraté, le lactate de Mn(II) trihydraté, le phosphate de Mn (II), l'iodure de Mn(II), le nitrate de Mn(II) trihydraté, le bromure de Mn(II), le perchlorate de Mn(II) tétrahydraté et le sulfate de Mn(II) monohydraté.

Les sels utilisés avantageusement sont $MnCl_2$ et $ZnCl_2$.

Les sels d'acides carboxyliques utilisables dans l'invention incluent également des sels d'acides carboxyliques hydroxylés tels que le gluconate.

Les sels de Mn(II) et Zn(II) peuvent être introduits sous forme solide dans le milieu physiologique ou bien provenir d'une eau naturelle, minérale ou thermale, riche en ces ions (par exemple sous forme d'hydroxydase) ou encore d'eau de mer (mer morte notamment). Ils peuvent aussi provenir d'argile (argile verte par exemple) ou même d'extrait végétal les contenant.

Parmi les hydrogénocarbonates alcalins et alcalino-terreux, on peut citer les hydrogénocarbonates de Na, K, Mg, Ca et leurs mélanges, et en particulier l'hydrogénocarbonate de Na. Ces hydrogénocarbonates (appelés aussi bicarbonates) peuvent provenir d'une eau naturelle, par exemple eau de source du bassin de Vichy, de La Roche Posay, eau de Badoit.

Comme indiqué précédemment, le système catalytique chimique utilisé selon l'invention constitue une pseudo-oxydase spécifique qui permet l'oxydation des orthodiphénols, en présence d'oxygène, comme le feraient certains catalyseurs enzymatiques naturels ayant une activité polyphénoloxydase. Il permet en outre, par oxydation du précurseur de densification, la coloration des fibres kératiniques. Ainsi, des cheveux gris peuvent être colorés en marron, ce qui renforce la perception de la densification.

Les précurseurs de densification des compositions de l'invention sont des composés ou mélanges de composés comprenant au moins un cycle aromatique, de préférence un cycle benzénique, comportant au moins deux groupes hydroxyles (OH) portés par deux atomes de carbone consécutifs du cycle aromatique.

Le cycle aromatique peut être un cycle aromatique condensé contenant éventuellement un ou plusieurs hétéroatomes, tel que le naphtalène, le tétrahydronaphtalène, l'indane, l'indène, l'anthracène, le phénanthrène, l'indole, l'isoindole, l'indoline, l'isoindoline, le benzofuranne, le dihydrobenzofuranne, le chromane, l'isochromane, le chromène,

l'isochromène, la quinoléine, la tétrahydroquinoléine et l'isoquinoléine.

A titre d'exemple, les précurseurs de densification selon l'invention peuvent être représentés par la formule (I) suivante :

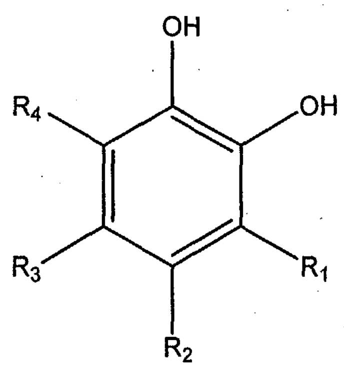

(I)

dans laquelle les substituants $R_1$ à $R_4$, identiques ou différents, représentent un atome d'hydrogène, un radical halogène, hydroxyle, carboxyle, carboxylate d'alkyle, amino éventuellement substitué, alkyle linéaire ou ramifié éventuellement substitué, alcényle linéaire ou ramifié éventuellement substitué, cycloalkyle éventuellement substitué, alcoxy, alcoxyalkyle, alcoxyaryle, le groupe aryle pouvant être éventuellement substitué, aryle, aryle substitué, un radical hétérocyclique éventuellement substitué, un radical hétérocyclique éventuellement substitué, un radical contenant un ou plusieurs atomes de silicium, où deux des substituants $R_1$ à $R_4$ forment conjointement un cycle saturé ou insaturé contenant éventuellement un ou plusieurs hétéroatomes et éventuellement condensé avec un ou plusieurs cycles saturés ou insaturés contenant éventuellement un ou plusieurs hétéroatomes.

Les cycles saturés ou insaturés, éventuellement condensés, peuvent être aussi éventuellement substitués.

Les radicaux alkyles sont généralement les radicaux alkyles en $C_1$-$C_{10}$, de préférence les radicaux alkyles en $C_1$-$C_6$, tels que méthyle, éthyle, propyle, butyle, pentyle et hexyle.

Les radicaux alcoxy sont en général les radicaux alcoxy en $C_1$-$C_{20}$, tels que méthoxy, éthoxy, propoxy et butoxy.

Les radicaux alcoxy alkyles sont de préférence les radicaux alcoxy ($C_1$-$C_{20}$) alkyle ($C_1$-$C_{20}$), tels que méthoxyméthyle, éthoxyméthyle, méthoxyéthyle, éthoxyéthyle, etc.

Les radicaux cycloalkyles sont en général les radicaux cycloalkyles en $C_4$-$C_{8,}$ de préférence les radicaux cyclopentyle et cyclohexyle. Les radicaux cycloalkyles peuvent être des radicaux cycloalkyles substitués, en particulier par des groupes alkyles, alcoxy, acide carboxylique, hydroxyle, amine et cétone.

Les radicaux alcényles sont de préférence des radicaux en $C_2$-$C_{20}$, tels que éthylène, propylène, butylène, pentylène, méthyl-2-propylène et décylène.

Les radicaux contenant un ou plusieurs atomes de silicium sont de préférence des radicaux polydiméthylsiloxane, polydiphénylsiloxane, polydiméthylphénylsiloxane, stéaroxydiméthicone.

Les radicaux hétérocycliques sont en général des radicaux comprenant un ou plusieurs hétéroatomes choisis parmi O, N et S, de préférence O ou N, éventuellement substitués par un ou plusieurs groupes alkyles, alcoxy, acide carboxylique, hydroxyle, amine ou cétone.

Parmi les radicaux hétérocycliques utilisables, on peut citer les groupes furyle, pyrannyle, pyrrolyle, imidazolyle, pyrazolyle, pyridyle, thiényle.

De préférence encore, les groupes hétérocycliques sont des groupes condensés tels que des groupes benzofurannyle, chromènyle, xanthényle, indolyle, isoindolyle, quinolyle, isoquinolyle, chromannyle, isochromannyle, indolinyle, isoindolinyle, coumarinyle, isocoumarinyle, ces groupes pouvant être substitués, en particulier par un ou plusieurs groupes OH.

Les précurseurs de densification utilisables dans l'invention sont en particulier:

- les flavonols comme la catéchine et le gallate d'épichatéchine,
- les flavonols comme la quercétine,
- les anthocyanidines comme la péonidine,
- les anthocyanines, par exemple l'oénine,
- les hydroxybenzoates, par exemple les sels d'acide gallique,
- les flavones comme la lutéoline,

- les iridoïles comme l'oleuropéine,

la lutéoline, l'oleuropéine et les anthocyanines pouvant être osylés (par exemple glucosylés) et/ou sous forme d'oligomères (procyanidines),

- les hydroxystilbènes, par exemple le tétrahydroxy-3,3',4,5'-stilbène, éventuellement oxylés (par exemple glucosylés),
- la 3,4-dihydroxyphénylalanine et ses dérivés,
- la 2,3-dihydroxyphénylalanine et ses dérivés,
- la 4,5-dihydroxyphénylalanine et ses dérivés,
- le 4,5-dihydroxyindole et ses dérivés,
- le 5,6-dihydroxyindole et ses dérivés,
- le 6,7-dihydroxyindole et ses dérivés,
- le 2,3-dihydroxyindole et ses dérivés,
- les dihydroxycinnamates tels que l'acide caféique et l'acide chlorogénique,
- les hydroxycoumarines,
- les hydroxyisocoumarines,
- les hydroxycoumarones,
- les hydroxyisocoumarones,
- les hydroxychalcones,
- les hydroxychromones,
- les anthocyanes,
- les quinones,
- les hydroxyxanthones,
- les 1,2 dihydroxybenzènes,
- les 1,2,4 trihydroxybenzènes,
- les 1,2,3 trihydroxybenzènes,
- le 2,4,5 trihydroxytoluène,
- la 5,6 dihydroxyindoline, et
- les mélanges des composés précédents.

Lorsque les précurseurs de densification présentent des formes D et L, les deux formes peuvent être utilisées dans les compositions selon l'invention, ainsi que les racémiques.
La quantité de précurseurs de densification dans la composition finale doit être suffisante pour obtenir une densification visible à l'oeil nu. Cette quantité peut varier dans de larges mesures en fonction de la nature du précurseur et de l'intensité voulue pour la densification.
En général, on obtient une densification convenable lorsque le ou les précurseurs de densification représente (nt) au moins 0,001% en poids du poids total de la composition. Il(s) peut (peuvent) représenter jusqu'à 80 % du poids total de la composition. En pratique il(s) représente(nt) de 0,01 % à 25 % et mieux de 2 % à 8 % du poids total de la composition.
En faisant varier la nature des différents précurseurs de densification et leurs proportions dans la composition, on peut faire varier la densification ainsi que la couleur des fibres kératiniques, après application de la composition sur lesdites fibres.
Par exemple, on peut utiliser un ratio 1/10 d'acide chlorogénique et de catéchine et obtenir une coloration marron des cheveux ou un ratio 1/1 et obtenir une coloration acajou.
Les précurseurs de densification peuvent être des extraits de plantes, fruits, agrumes, légumes, arbres, arbustes et des mélanges de ces extraits, qui contiennent de nombreux polyphénols tels que définis précédemment.
Parmi les extraits de plantes, on peut citer les extraits de rose, de sorgho et de thé.
Parmi les extraits de fruits, on peut citer les extraits de pomme, de raisin (en particulier de pépins de raisin), de cacaotier (fèves et/ou cabosses) et de banane.
Parmi les extraits de légumes, on peut citer l'extrait de pomme de terre.
Parmi les extraits d'arbres, on peut citer les écorces de pin.
On peut également utiliser des mélanges d'extraits de plantes et/ou de fruits tels que des mélanges d'extraits de pomme et de thé et des mélanges d'extraits de raisin et de pomme.
Suivant les parties de fruits utilisés, par exemple pulpe ou pépins de raisin, la densification obtenue est différente.
En particulier, le précurseur de densification est choisi parmi les extraits de thé, de raisin, de pomme, de banane, de cacaotier, de sorgho, de pomme de terre et leurs mélanges.

La composition de l'invention peut être à usage cosmétique ou pharmaceutique. Préférentiellement, la composition de l'invention est à usage cosmétique. Aussi, la composition doit contenir un milieu physiologiquement acceptable non toxique et susceptible d'être appliqué sur la peau et les phanères d'êtres humains. Par "cosmétique", on entend au sens de l'invention une composition d'aspect, d'odeur et de toucher agréables.

Le milieu physiologiquement acceptable est un milieu solide ou liquide ne nuisant pas à la propriété de densification des précurseurs ni à l'effet catalytique du système catalytique. Avantageusement, il facilite la polymérisation « in situ » des orthodiphénols, ce qui lui confère un effet bactériostatique.

Cette composition peut se présenter sous toutes formes galéniques connues adaptées à une application directe sur le cuir chevelu et/ou sur les cheveux (du type application topique).

Pour une application topique sur la peau, la composition peut avoir la forme d'une solution ou suspension aqueuse, alcoolique ou hydro-alcoolique ou d'une suspension huileuse, d'une émulsion de consistance plus ou moins fluide et notamment liquide ou semi-liquide, obtenue par dispersion d'une phase grasse dans une phase aqueuse (H/E) ou inversement (E/H), d'une émulsion solide (H/E) ou (E/H), d'un gel aqueux, hydro-alcoolique ou huileux plus ou moins fluide ou solide, d'une poudre libre ou compactée à utiliser telle quelle ou à incorporer dans un milieu physiologiquement acceptable, ou encore de microcapsules ou microparticules, de dispersions vésiculaires de type ionique et/ou non ionique. Elle peut ainsi se présenter sous forme d'une lotion, sérum, lait, crème H/E ou E/H, d'onguent, pommade, baume, patch, tampon imbibé.

On peut également envisager une composition sous forme de mousse ou encore sous forme de spray ou d'aérosol comprenant alors un agent propulseur sous pression.

En particulier la composition à application sur le cuir chevelu ou les cheveux peut se présenter sous forme d'une lotion de soin capillaire, par exemple d'application journalière ou bi-hebdomadaire, d'un shampooing ou d'un après-shampooing capillaire, en particulier d'application bi-hebdomadaire ou hebdomadaire, d'un savon liquide ou solide de nettoyage du cuir chevelu d'application journalière, d'un produit de mise en forme de la coiffure (laque, produit pour mise en pli, gel coiffant), d'un masque traitant, d'une crème ou d'un gel moussant de nettoyage des cheveux. Elle peut encore se présenter sous forme de teinture ou de mascara capillaire à appliquer au pinceau ou au peigne.

Selon un mode de réalisation particulier, la composition selon l'invention se présente sous forme de crème ou lotion capillaire, de shampooing ou d'après-shampooing capillaire.

Les quantités des différents constituants du milieu physiologique de la composition selon l'invention sont ceux généralement utilisées dans les domaines considérés. En outre, ces compositions sont préparées selon les méthodes usuelles.

Lorsque la composition est une émulsion, la proportion dé la phase grasse peut aller de 2 % à 80 % en poids, et de préférence de 5 % à 50 % en poids par rapport au poids total de la composition. La phase aqueuse est ajustée en fonction de la teneur en phase grasse et en composé(s) (I) ainsi que de celle des éventuels ingrédients additionnels, pour obtenir 100 % en poids. En pratique la phase aqueuse représente de 5 % à 99,9 % en poids.

La phase grasse peut contenir des composés gras ou huileux, liquides à température ambiante (25°C) et pression atmosphérique (760 mm de Hg), généralement appelés huiles. Ces huiles peuvent être compatibles ou non entre elles et former une phase grasse liquide macroscopiquement homogène ou un système bi- ou triphasique.

La phase grasse peut, en plus des huiles, contenir des cires, des gommes, des polymères lipophiles, des produits "pâteux" ou visqueux contenant des parties solides et des parties liquides.

Le milieu physiologiquement acceptable est de préférence un milieu comprenant une phase solubilisant les précurseurs de densification mais aussi le système catalytique. Par solubilisant, on entend selon l'invention qu'au moins 0,005 % de chacun des constituants de l'invention sont dissous dans le milieu.

Parmi les solvants des précurseurs de densification convenant pour la formulation des compositions utilisables dans l'invention, on peut citer l'eau, les alcools aliphatiques mono- ou dihydroxylés et les polyols, les éthers de polyols et leurs mélanges. Les alcools peuvent être des alcools aliphatiques mono- ou dihydroxylés. On peut aussi utiliser des alcools aromatiques.

Parmi ces solvants, on peut citer à titre d'exemple, les alcanols inférieurs en $C_1$-$C_8$ et mieux $C_1$-$C_4$ à un groupe hydroxyle comme l'éthanol et l'isopropanol ; les alcanediols comme l'éthylèneglycol ou le butanediol-1,4, le propylèneglycol ; les polyols ou éthers de glycols, comme le 2 butoxyéthanol, la glycérine, le sorbitol, la diglycérine, le monoéthyléther et le monométhyléther de diéthylèneglycol ; l'acétone ainsi que les alcools aromatiques comme l'alcool benzylique ou le phénoxyéthanol et les produits analogues comme le 1-phénoxy-2-propanol ou leurs mélanges.

Les solvants sont présents de préférence dans des proportions allant de 1 à 80 % en poids, et en particulier de 5 à 60 % en poids par rapport au poids total de la composition, par exemple de 10 % à 30 % en poids.

Les alcools sont avantageusement choisis parmi les alcanols notamment inférieurs ($C_1$-$C_4$), tels que l'éthanol et l'isopropanol et les alcanediols notamment en C2-C6 tels que le propylène glycol et le pentane diol.

Le milieu physiologiquement acceptable comprend de préférence de l'eau (en particulier distillée ou permutée) ou un mélange eau/alcool, en particulier eau/éthanol.

La quantité d'alcool dans le mélange eau/alcool peut représenter jusqu'à 80% en poids du mélange eau/alcool, de préférence 1 à 50% en poids et mieux 5 à 20 % en poids.

La composition utilisable dans l'invention peut en outre contenir, au moins un agent tensioactif représentant au moins 0,01% du poids total de la composition. Il représente en pratique de 0,1 % à 40 % du poids total de la composition, par exemple de 0,5 à 30% en poids et mieux de 10 % à 20 % en poids.

Les agents tensioactifs peuvent être choisis parmi les agents tensioactifs anioniques, cationiques, non-ioniques, amphotères ou leurs mélanges, et de préférence parmi les tensioactifs non ioniques. Ces agents tensioactifs sont en particulier ceux utilisés dans les shampoings et après-shampoings pour cheveux humains, ayant un effet nettoyant.

Parmi ces agents tensioactifs, on peut citer les alkylbenzène sulfonates, les alkylnaphtalène sulfonates, les sulfates, les éthers sulfates et les sulfonates d'alcools gras (d'au moins 12 atomes de carbone), les sels d'ammonium quaternaires, tels que le bromure de triméthylcétylammonium ou le bromure de cétylpyridinium, les éthanolamides d'acides gras éventuellement oxyéthylénés, les acides, les alcools ou les amines polyoxyéthylénés, les alcools polyglycérolés, les alkylphénols polyoxyéthylénés ou polyglycérolés, ainsi que les alkylsulfates polyoxyéthylénés.

Lorsque la composition selon l'invention se présente sous forme d'émulsion, elle contient en particulier un ou plusieurs émulsionnants et/ou co-émulsionnants tels que ceux généralement utilisés dans les domaines cosmétique et pharmaceutique. Leur nature est, en outre, fonction du sens de l'émulsion. En pratique, l'émulsionnant et éventuellement le co-émulsionnant sont présents, dans la composition, en une proportion allant de 0,1 % à 30 % en poids, de préférence de 0,5 à 20 % en poids et mieux de 1 à 8%. L'émulsion peut, en outre, contenir des vésicules lipidiques et notamment des liposomes.

Comme émulsionnants utilisables dans l'invention, on peut citer par exemple le stéarate ou laurate de glycérol, les stéarates ou oléates de sorbitol, les alkyl diméthiconecopolyol (avec alkyle $\geq$ 8) et leurs mélanges pour une émulsion E/H. On peut aussi utiliser le monostéarate ou monolaurate de polyéthylène glycol, le stéarate ou oléate de sorbitol polyoxyéthyléné, les diméthiconecopolyols et leurs mélanges pour une émulsion H/E.

La composition de l'invention peut comprendre, en outre, d'autres ingrédients usuellement utilisés dans les domaines concernés, choisis parmi les épaississants ou gélifiants de phase hydro-alcoolique ou de phase huileuse, les matières colorantes solubles dans le milieu de la composition, les particules solides du type charges ou pigments, les antioxydants, les conservateurs, les parfums, les électrolytes, les neutralisants, les agents bloqueurs d'U.V. comme les filtres solaires, les actifs cosmétiques et pharmaceutiques, leurs mélanges. Ces ingrédients additionnels peuvent être présents dans la composition selon les quantités généralement utilisées dans le domaine cosmétique et dermatologique et notamment à raison de 0,01 à 50% du poids total de la composition et mieux de 0,1 à 20% et par exemple de 0,1 à 10 %.

Comme gélifiants ou épaississants hydroalcooliques utilisables dans l'invention, on peut citer les polymères carboxyvinyliques (carbomer), les copolymères acryliques tels que les copolymères d'acrylates/alkylacrylates et les polymères d'acide acrylique, les polyacrylamides, les polysaccharides tels que l'hydroxypropylcellulose ou l'hydroxyéthylcellulose, les gommes naturelles et les argiles comme la gomme arabique, la gomme de xanthane, l'alginate de sodium, leurs mélanges et, comme gélifiants ou épaississants lipophiles, on peut citer les argiles modifiées comme les Bentones, les sels métalliques d'acides gras comme les stéarates d'aluminium, la silice traitée hydrophobe, l'éthylcellulose, leurs mélanges.

Ces gélifiants ou épaississants sont présents en pratique dans des proportions allant de 0,1 à 5% et en particulier de 0,2 à 3% en poids par rapport au poids total de la composition.

Bien entendu, l'homme du métier veillera à choisir le ou les éventuels ingrédients additionnels mentionnés précédemment, de manière telle que les propriétés avantageuses attachées intrinsèquement à la composition de densification selon l'invention ne soient pas, ou substantiellement pas, altérées par la ou les adjonctions envisagées. En particulier, les antioxydants et les conservateurs présents dans la composition sont tels qu'ils ne doivent pas interférer avec la réaction de densification, et notamment ne pas réagir avec les sels et oxydes de Mn(II) ou Zn(II), les hydrogénocarbonates et les précurseurs de densification, tels que les polyphénols ayant un motif de répétition du type orthophénol.

La composition selon l'invention peut contenir un ou plusieurs agents de chélation des ions du système catalytique, à condition que ces agents soient en une quantité telle qu'il subsiste dans la composition des ions Mn(II) et/ou Zn(II) en quantité suffisante pour catalyser l'oxydation du composé de densification. Autrement dit, la quantité molaire d'agent de chélation doit être inférieure à celle des ions Mn(II) et/ou Zn(II) présents dans la composition.

De façon avantageuse, la composition selon l'invention est exempte d'agents de chélation des sels de Mn(II) et/ou Zn(II) utilisés, car ces agents tendent à inhiber l'oxydation des précurseurs de densification.

La composition utilisable dans l'invention a en particulier un pH qui varie de 7 à 12, de préférence de 7 à 9, et typiquement de l'ordre de 8. Ce pH, proche de la neutralité permet d'utiliser le procédé de densification selon l'invention sur des personnes à cuir chevelu sensible. Ce pH est ajusté par l'utilisation d'agents alcalinisants ou acidifiants bien connus de l'état de la technique pour le traitement des fibres kératiniques humaines.

Parmi les agents alcalinisants on peut citer, à titre d'exemple, l'ammoniaque, les carbonates alcalins, les

alcanolamines telles que les mono-, di- et triéthanolamines ainsi que leurs dérivés, les hydroxydes de sodium ou de potassium et les composés de formule (V) suivante :

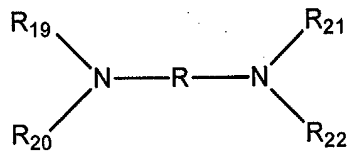
(V)

dans laquelle :

- R est un reste propylène éventuellement substitué par un groupement hydroxyle ou un radical alkyle en $C_1$-$C_4$ ; et
- $R_{19}$, $R_{20}$, $R_{21}$ et $R_{22}$, identiques ou différents, représentent un atome d'hydrogène, un radical alkyle en $C_1$-$C_4$ ou hydroxyalkyle en $C_1$-$C_4$.

Les agents acidifiants utilisables dans l'invention, dans le cas par exemple d'une composition à deux compartiments et pour stabiliser le mélange orthodiphénol + Mn(II) et/ou Zn(II), la quantité de bicarbonate du deuxième compartiment sera ajustée pour avoir un pH supérieur à 7, au moment de l'application. A à titre d'exemple, on peut utiliser les acides minéraux ou organiques comme l'acide chlorhydrique, l'acide orthophosphorique, les acides carboxyliques comme l'acide citrique, l'acide lactique, ou les acides sulfoniques.

L'effet densificateur peut être amélioré en utilisant des acides organiques ou minéraux tels que décrits précédemment après que la réaction catalytique se fut produite sur le cuir chevelu. La rémanence de l'effet (en particulier la tenue de la densification après plusieurs shampoings) est augmentée par les propriétés de fixation des acides. Par ailleurs, la modification du pH, suite à l'action d'un acide, peut modifier l'effet densificateur et éventuellement l'atténuer. Dans ce cas, l'application d'une base minérale ou organique augmentera l'intensité de l'effet densificateur. Ces modulations de l'effet de densification permettent d'adapter cet effet en fonction du souhait de l'utilisateur.

Comme huiles utilisables dans l'invention, on peut citer les huiles d'origine minérale (huile de vaseline, isoparaffine hydrogénée), les huiles d'origine végétale (fraction liquide du beurre de karité, huile de tournesol, d'abricot, alcool ou acide gras), les huiles d'origine animale (perhydrosqualène), les huiles de synthèse (ester d'acide gras, huile de Purcellin), les huiles siliconées (polydiméthylsiloxane linéaire ou cyclique, phényltriméthicone) et les huiles fluorées (perfluoropolyéthers). Comme cires, on peut citer les cires siliconées, les cires d'abeille, de carnauba ou paraffine, de polyéthylène.

La composition peut contenir, en outre, un actif cosmétique ou dermatologique additionnel qui peut être hydrophile et choisi parmi les protéines ou les hydrolysats de protéine, les acides aminés, les polyols, l'urée, l'allantoïne, les sucres et les dérivés de sucre, les vitamines hydrosolubles, les extraits végétaux différents de ceux décrits précédemment (par exemple ceux d'Iridacées ou de soja), les sels ferriques ou ferreux et les hydroxy-acides ; ou lipophile et choisi parmi le rétinol (vitamine A) et ses dérivés notamment ester (palmitate de rétinol), le tocophérol (vitamine E) et ses dérivés notamment ester (acétate de tocophérol), les acides gras essentiels, les céramides, les huiles essentielles, l'acide salicylique et ses dérivés comme le n-octanoyl-5 salicylique, les esters des hydroxy-acides, les phospholipides comme la lécithine, leurs mélanges.

La composition selon l'invention permet une densification immédiate des fibres kératiniques, et en particulier des cheveux. Ainsi, après 3 minutes d'application par voie topique de la composition selon l'invention sur les fibres et la peau d'où émergent les fibres, et plus spécialement sur les cheveux et le cuir chevelu, on observe à l'oeil nu un effet de densification, effet qui prend toute son amplitude dans les 8-15 minutes suivant l'application, voire au bout de 12-72 heures selon le milieu physiologique utilisé.

Selon un mode particulier de réalisation de l'invention, on peut coupler l'action de densification immédiate de l'association précurseur de densification + système catalytique, à l'action de composés additionnels augmentant la densité pilaire et/ou favorisant la repousse des fibres kératiniques notamment des cheveux et/ou limitant la chute de ces fibres (cheveux en particulier). Ces composés actifs additionnels peuvent être à action lente ou retardée, en vue de se substituer dans le temps à l'association selon l'invention à effet immédiat. Ces composés actifs additionnels sont ceux connus par l'homme du métier pour avoir un effet sur la densité pilaire.

Avantageusement, ces composés actifs à effet retardé augmentant la densité pilaire et/ou favorisant la repousse des fibres kératiniques et/ou limitant leur chute, appelés ci-après actifs antichute/repousse à effet retardé, sont des actifs à application topique, appliqués localement sur les fibres (cheveux notamment) et/ou la peau d'où émergent ces fibres (cuir chevelu notamment).

Aussi, l'invention a encore pour objet une composition de soin des fibres kératiniques humaines et/ou de la

peau d'où émergent ces fibres, et plus spécialement une composition de soin des cheveux et/ou du cuir chevelu, contenant dans un milieu physiologiquement acceptable, a) une quantité efficace d'au moins un précurseur de densification choisi parmi les composés comportant au moins un cycle aromatique ayant au moins deux groupes hydroxyles (OH) portés par deux atomes de carbone consécutifs du cycle aromatique, b) une quantité efficace d'un système catalytique comprenant un premier constituant choisi parmi les sels et oxydes de Mn(II), et/ou Zn(II) et leurs mélanges et un second constituant choisi parmi les hydrogénocarbonates alcalins, les hydrogénocarbonates alcalino-terreux et leurs mélanges, et c) une quantité efficace d'au moins un actif à effet retardé augmentant la densité pilaire et/ou favorisant la repousse des fibres kératiniques notamment des cheveux et/ou limitant la chute de ces fibres et en particulier des cheveux, les proportions du premier constituant et du second constituants étant telles que :

$$\frac{[Mn(II)]}{[HCO_3]} \leq 1 \text{ avec } [\mathrm{Mn(II)}] \neq 0$$

$$[\frac{Zn(II)]}{[HC0_3]} \leq 1 \text{ avec } [\mathrm{Zn(II)}] \neq 0$$

$$\frac{[Mn(II) + Zn(II)]}{[HCO_3]} \leq 1 \text{ avec } [\mathrm{Mn(II)}] \text{ et } [\mathrm{Zn(II)}] \neq 0$$

où [Mn(II)], [Zn(II)] et [$HCO_3$] représentent respectivement les concentrations molaires en Mn(II), Zn(II) et $HCO_3$ dans la composition.

Avantageusement, les composés additionnels actifs à effet retardé sont notamment choisis parmi les inhibiteurs de lipoxygénase tels que décrits dans EP 0648488 et notamment les dérivés des butanes catécholiques comme l'acide nordihydroguaiarétique (NDGA) et son énantiomère le masoprocol, les inhibiteurs de bradykinine décrits notamment dans EP 0845700, les prostaglandines et leurs dérivés notamment ceux décrits dans WO 98/33497, WO 95/11003, JP 97-100091, JP 96-134242, les agonistes ou antagonistes des récepteurs des prostaglandines, les analogues non prostanoïques de prostaglandines tels que décrits dans EP 1175891 et EP 1175890, WO 01/74307, WO 01/74313, WO 01/74314, WO 01/74315 ou WO 01/72268, leurs mélanges.

Comme autres composés additionnels favorisant la pousse du cheveu pouvant être présents dans la composition selon l'invention on peut citer les vasodilatateurs, les antiandrogènes, les cyclosporines et leurs analogues, les anti-microbiens et antifongiques, les anti-inflammatoires, les rétinoïdes, seuls ou en mélange.

Les vasodilatateurs utilisables sont notamment les agonistes des canaux potassium incluant le minoxidil ainsi que les composés décrits dans les brevets US 3 382247, 5 756092, 5 772990, 5 760043, 5 466694, 5 438058, 4 973474, la cromakalim, le nicorandil et le diaxozide, seuls ou en association.

Les anti-androgènes utilisables incluent notamment les inhibiteurs stéroïdiens ou non stéroïdiens de 5$\alpha$-réductase, comme le finastéride et les composés décrits dans US 5 516779, l'acétate de cyprostérone, l'acide azélaïque, ses sels et ses dérivés et les composés décrits dans US 5 480913, le flutamide et les composés décrits dans les brevets US 5 411981, 5 565467 et 4 910226.

Les composés anti-microbiens ou antifongiques peuvent être choisis parmi les dérivés du sélénium, le kétoconazole, l'octopirox, le triclocarban, le triclosan, le pyrithione zinc, l'itraconazole, l'acide asiatique, l'hinokitiol, la mipirocine, les tétracyclines, notamment l'érythromycine et les composés décrits dans EP 0680745, le chlorhydrate de clinycine, le peroxyde de benzoyle ou de benzyle et la minocycline.

Les anti-inflammatoires peuvent être choisis parmi les anti-inflammatoires stéroïdiens comme les glucocorticoïdes, les corticostéroïdes (par exemple : l'hydrocortisone) et les anti-inflammatoires non stéroïdiens comme l'acide glycyrrhétinique et l'$\alpha$-bisabolol, la benzydamine, l'acide salicylique et les composés décrits dans EP 0770399, WO 94/06434 et FR 2268523.

Les rétinoïdes peuvent être choisis parmi l'isotrétinoïne, l'acitrétine et le tazarotène.

Comme autres composés actifs pour favoriser la pousse et/ou limiter la chute des cheveux à effet retardé utilisables en associations avec le précurseur de densification et le système catalytique décrits précédemment, on peut citer l'aminexil, le 6-0-[(9Z,12Z)-octadéca-9,12-diènoyl]hexapyranose, le chlorure de benzalkonium, le chlorure de benzéthonium, le phénol, l'oestradiol, le maléate de chlorphéniramine, les dérivés de chlorophylline, le cholestérol, la cystéine, la méthionine, le menthol, l'huile de menthe poivrée, le panthoténate de calcium, le panthénol, le résorcinol, les activateurs de la protéine kinase C, les inhibiteurs de la glycosidase, les inhibiteurs de glycosaminoglycanase, les esters d'acide pyroglutamique, les acides hexosaccharidiques ou acyl-hexosaccharique, les éthylènes aryl substitués, les amino-acides N-acylés, les flavonoïdes, les dérivés et analogues d'ascomycine, les antagonistes d'histamine, les

saponines, les inhibiteurs de protéoglycanase, les agonistes et antagonistes d'estrogènes, les pseudotèrines, les cytokines et les promoteurs de facteurs de croissance, les inhibiteurs d'IL-1 ou d'IL-6, les promoteurs d'IL-10, les inhibiteurs de TNF, les hydroxy-acides, les benzophénones et l'hydantoïne, l'acide rétinoïque ; les vitamines comme la vitamine D, les analogues de la vitamine B12 et le panthoténol ; les triterpènes comme l'acide ursolique et les composés décrits dans US 5529769, US 5468888, US 5631282 ; les agents antiprurigineux comme la thénaldine, la triméprazine ou la cyproheptadine ; les antiparasitaires, en particulier le métronidazole, le crotamiton ou les pyréthrinoïdes ; les antifongiques, en particulier l'octopirox et les composés appartenant à la classe des imidazoles tels que l'éconazole, le kétoconazole ou le miconazole ou leurs sels, les esters d'acide nicotinique, dont notamment le nicotinate de tocophérol, le nicotinate de benzyle et les nicotinates d'alkyles en $C_1$-$C_6$ comme les nicotinates de méthyle ou d'hexyle ; les donneurs de NO comme le nitroprussiate de sodium dihydraté ou la nitrosoglutathione ; les inhibiteurs de guanylate-cyclase comme le sildénafil (Viagra®), les agents antagonistes de calcium, comme la cinnarizine, le diltiazem, la nimodipine, vérapamil, l'alvérine et la nifédipine ; les hormones telles que l'estriol ou ses analogues, la thyroxine et ses sels, la progestérone ; les agents antiandrogènes tels que l'oxendolone, la spironolactone, le diéthylstilbestrol et la flutamide ; les inhibiteurs de métalloprotéinase (MMP9) tels que décrits dans FR 2778558 ; leurs mélanges.

En particulier, l'actif à effet retardé est choisi parmi les inhibiteurs de lipoxygénases, et notamment parmi les dérivés des butanes catécholiques comme l'acide nordihydroguaiarétique (NDGA) et son énantiomère le masoprocol, et les vasodilatateurs et notamment parmi les agonistes des canaux potassium incluant le minoxidil, leurs associations.

La quantité efficace d'un actif à effet retardé correspond à la quantité nécessaire pour obtenir le résultat désiré (à savoir augmenter la densité des cheveux). L'homme du métier est donc en mesure d'évaluer cette quantité efficace qui dépend de la nature du composé utilisé, de la personne à laquelle on l'applique, et du temps de cette application.

Pour donner un ordre de grandeur, selon l'invention, le composé actif en antichute/repousse à effet retard peut être utilisé, pour application topique, en une quantité représentant de $10^{-4}$ % à 8% du poids total de la composition et mieux en une quantité représentant de $10^{-2}$ % à 5% du poids total de la composition, par exemple de 0,5 à 3 %.

Il est aussi possible selon l'invention de coupler l'association précurseur de densification + système catalytique à des actifs antichute/repousse utilisés par voie orale comme le finastéride et /ou à des supplémentassions alimentaires notamment en fer et/ou acides aminés comme la cystine et la taurine. En pratique, la quantité d'actifs antichute/repousse utilisée par voie orale peut représenter de 0,1 à 300 mg par jour, par exemple de 5 à 10mg/j.

Le procédé de densification selon l'invention peut être appliqué sur des fibres kératiniques humaines telles que les cheveux, les cils, les sourcils et les poils et plus spécialement sur les cheveux.

Pour la densification des fibres kératiniques, différents procédés d'application selon l'invention peuvent être utilisés.

Selon un premier procédé, on applique sur les fibres kératiniques, en présence d'oxygène, par exemple l'oxygène de l'air, une composition comprenant tous les ingrédients de la composition de l'invention.

Selon un deuxième procédé, on peut en premier lieu appliquer sur les fibres kératiniques une première composition d'un ou plusieurs précurseurs de densification dans un milieu physiologiquement acceptable, puis sur cette première composition, une deuxième composition contenant le système catalytique dans un milieu physiologiquement acceptable. On peut bien évidemment inverser l'ordre d'application de ces compositions.

L'application des compositions peut se faire par tout moyen connu, en particulier par pulvérisation.

Les compositions selon l'invention peuvent se présenter et être conditionnées sous différentes formes.

Selon un premier mode de réalisation, la composition utilisable pour l'invention peut être conditionnée sous forme d'aérosol à un seul compartiment dans lequel se trouve la composition renfermant le ou les précurseurs de densification, le système catalytique et un gaz propulseur inerte classique tel que de l'azote, un hydrocarbure saturé comme l'isopropane ou un hydrocarbure halogéné.

Dans un seconde mode de réalisation, la composition utilisable pour la mise en oeuvre de l'invention peut être conditionnée sous forme d'un kit comportant deux conteneurs distincts, l'un pour la composition de base contenant le ou les précurseurs de densification, l'autre pour le système catalytique, la composition de base et le système catalytique étant mélangés ou appliqués successivement au moment de l'emploi.

Dans une troisième mode de réalisation, la composition peut être contenue dans un système à pompe à un seul compartiment, sans reprise d'air, ou dans un système à pompe à deux compartiments, le précurseur de densification étant dans un compartiment et le système catalytique dans l'autre compartiment.

Dans un quatrième mode de réalisation, la composition contient dans un premier compartiment une première solution de sel(s) et/ou oxyde(s) de Mn(II) et/ou de Zn(II) et d'un ou plusieurs orthodiphénols et dans un autre compartiment une deuxième solution contenant le bicarbonate ; l'actif antichute/repousse à effet retard étant présent dans l'un ou l'autre des compartiments.

La composition selon l'invention peut être appliquée sur les zones alopéciques du cuir chevelu et des cheveux d'un individu, et éventuellement laissée en contact plusieurs heures et éventuellement rincée.

On peut, par exemple, appliquer la composition contenant une quantité efficace d'un composé de formule (I), salifié ou non, le soir, garder celle-ci au contact toute la nuit et éventuellement effectuer un shampooing le matin. Ces

applications peuvent être renouvelées quotidiennement pendant un ou plusieurs mois suivant les individus.

On va maintenant donner à titre d'illustration des exemples de réalisation de l'invention qui ne sauraient limiter en aucune façon sa portée.

**Exemple 1 : lotion de densification immédiate**

| Compositions | quantité et mode d'emploi |
|---|---|
| Propylène Glycol/éthanol/$H_2O$ (33/33/qsp 100)<br>Mn 1mM (55mg/l),<br>Propyl gallate 3%/NDGA** 1%/cathéchine 0,5% | Solution 1* à Appliquer sur cheveux et scalp |
| Sodium bicarbonate 1M dans $H_2O$ | Solution 2* (révélateur de densification) |

**NDGA représente l'acide nordihydroguaiarétique.

Les solutions 1 et 2 ont été appliquées successivement sur un cuir chevelu alopécique et des cheveux de couleur "poivre et sel" naturelle. On a laissé agir les formulations sur les cheveux pendant une heure puis on les a rincés à l'eau chaude du robinet.

Dès 3 minutes d'application, un effet de densification est perceptible à l'oeil nu, qui prend toute son amplitude dans les 8-15 minutes après application.

Ceci ressort clairement des photos annexées. La photo de gauche correspond au temps t=0 , juste avant application des solutions 1 puis 2, et la photo de droite correspond à t=8 min, après l'application.

**Exemple 2 : lotion cosmétique de densification immédiate incorporant un actif anti-chute du cheveu, à effet retardé**

| Compositions | Posologie et mode d'emploi |
|---|---|
| Propylène Glycol/éthanol/$H_2O$ (33/33/qsp 100)<br>Mn 1mM (55mg/l),<br>Propyl gallate 3%/NDGA 1%/cathéchine 0,5%<br>Aminexil 1,5% | Solution 1 à Appliquer sur cheveux et scalp |
| Sodium bicarbonate 1M dans $H_2O$ (permutée) | Solution 2 (révélateur de densification) |

**Exemple 3 : lotion pharmaceutique de densification immédiate incorporant un actif stimulant la repousse du cheveu à effet retardé**

| Compositions | Posologie et mode d'emploi |
|---|---|
| Propylène Glycol/éthanol/$H_2O$ (33/33/qsp 100)<br>Mn 1mM (55mg/l),<br>Propyl gallate 3%/NDGA 1%/cathéchine 0,5%<br>Minoxidil 2 % | Solution 1 à Appliquer sur cheveux et scalp |
| Sodium bicarbonate 1M dans $H_2O$ | Solution 2 (révélateur de densification) |

Les compositions des exemples 2 et 3 ont aussi été testées. Elles ont ainsi été appliquées successivement sur des cheveux blancs et pigmentés ainsi que sur le cuir chevelu. Ensuite, on les a laissées agir sur les cheveux pendant une heure puis les cheveux traités ont été rincés à l'eau chaude du robinet. Sur 9 personnes ayant testé ces compositions, 5 ont eu une impression immédiate de redensification et 4 ont eu une légère impression de redensification immédiate.

L'association d'orthodiphénols avec le système catalytique confère une bonne densification des cheveux. Cette densification résiste, en outre, à plusieurs shampoings.

Enfin, le pH proche de la neutralité pour ces formulations permet d'envisager une utilisation du procédé de l'invention même pour des cheveux de personnes ayant un cuir chevelu sensible.

En faisant varier la quantité d'orthodiphénols utilisée et le type de molécule utilisé, la densification peut être adaptée en fonction du phototype (en particulier couleur de la peau et des cheveux) du consommateur.

**EXEMPLE 4: Coiffage**

On réalise par simple mélange (à l'abri de l'air pour la composition 1) les compositions suivantes :

| | **COMPOSITIONS** | |
|---|---|---|
| | **1** | **A (comparative)** |
| L-DOPA | 1 g | - |
| $MnCl_2$ | 0,2 g | 0,2 g |
| $NaHCO_3$ | 1 g | 1 g |
| Eau | 50 ml | 50 ml |
| Ethanol | 50 ml | 50 ml |

Les deux compositions sont introduites dans un récipient que l'on munit d'un système de pulvérisation de type flacon pompe.

**Application et Evaluation :**

On prépare deux perruques de 15 g de cheveux naturels. Les cheveux sont maintenus aux racines sur bande de caoutchouc et laissés libres sur le reste de la longueur.

Les compositions sont pulvérisées sur les perruques (2 g par perruque). Les perruques sont laissées ainsi jusqu'au séchage.

On note les propriétés cosmétiques et en particulier, l'effet coiffant. Puis, les cheveux sont lavés avec un shampooing au lauryléther de sulfate de sodium, puis resséchés. On note une seconde fois les propriétés cosmétiques.

| Composition | Effet coiffant avant lavage | Effet coiffant après lavage |
|---|---|---|
| 1 | 30 | 25 |
| A | 30 | 0 |

Les résultats montrent que l'application d'une composition selon l'invention sur des cheveux, leur confère un effet coiffant résistant au lavage.

**Revendications**

1. Utilisation d'une quantité efficace d'au moins un précurseur de densification, choisi parmi les composés comportant au moins un cycle aromatique ayant au moins deux groupes hydroxyles portés par deux atomes de carbone consécutifs du cycle aromatique, et une quantité efficace d'un système catalytique comprenant un premier constituant choisi parmi les sels et oxydes de Mn(II) et/ou de Zn(II) et leurs mélanges et un second constituant choisi parmi les hydrogénocarbonates alcalins, les hydrogénocarbonates alcalino-terreux et leurs mélanges, dans une composition physiologiquement acceptable à application topique, comme agent pour augmenter la densité des fibres kératiniques humaines et notamment des cheveux ou pour le coiffage des cheveux, les proportions du premier et du second constituants étant telles que:

$$\frac{[Mn(II)]}{[HCO_3]} \leq 1 \text{ avec } [Mn(II)] \neq 0$$

$$\frac{[Zn(II)]}{[HC0_3]} \leq 1 \text{ avec } [Zn(II)] \neq 0$$

$$\frac{[Mn(II) + Zn(II)]}{[HCO_3]} \leq 1 \text{ avec } [Mn(II)] \text{ et } [Zn(II)] \neq 0$$

où [Mn(II)], [Zn(II)] et [$HCO_3$] représentent respectivement les concentrations molaires en Mn(II), Zn(II) et $HCO_3$ dans la composition.

2. Utilisation cosmétique d'une quantité efficace d'au moins un précurseur de densification, choisi parmi les composés comportant au moins un cycle aromatique ayant au moins deux groupes hydroxyles portés par deux atomes de carbone consécutifs du cycle aromatique, et une quantité efficace d'un système catalytique comprenant un premier constituant choisi parmi les sels et oxydes de Mn(II) et/ou de Zn(II) et leurs mélanges et un second constituant choisi parmi les hydrogénocarbonates alcalins, les hydrogénocarbonates alcalino-terreux et leurs mélanges, dans une composition cosmétique de soin capillaire d'être humain pour augmenter la densité des cheveux et/ou traiter l'alopécie androgénique ou pour le coiffage des cheveux.

3. Utilisation d'une quantité efficace d'au moins un précurseur de densification, choisi parmi les composés comportant au moins un cycle aromatique ayant au moins deux groupes hydroxyles portés par deux atomes de carbone consécutifs du cycle aromatique, et une quantité efficace d'un système catalytique comprenant un premier constituant choisi parmi les sels et oxydes de Mn(II) et/ou de Zn(II) et leurs mélanges et un second constituant choisi parmi les hydrogénocarbonates alcalins, les hydrogénocarbonates alcalino-terreux et leurs mélanges, pour la fabrication d'une composition capillaire d'être humain, destinée à augmenter la densité des cheveux et/ou traiter l'alopécie androgénique ou pour le coiffage des cheveux.

4. Utilisation selon l'une quelconque des revendications précédentes, **caractérisée en ce que** le rapport $\frac{[Mn(II)]}{[HCO_3]}$ est choisi dans la gamme allant de $10^{-5}$ à $10^{-1}$, de préférence de $10^{-3}$ à $10^{-2}$ et mieux est égal à $5.10^{-3}$.

5. Utilisation selon l'une quelconque des revendications 1 à 3, **caractérisée en ce que** le rapport $\frac{[Zn(II)]}{[HCO_3]}$ est choisi dans la gamme allant de $10^{-4}$ à < 1, de préférence de $10^{-3}$ à < 1, et mieux est de l'ordre de $5.10^{-1}$.

6. Utilisation selon l'une quelconque des revendications précédentes, **caractérisée en ce que** le rapport $\frac{[Mn(II) + Zn(II)]}{[HCO_3]}$ est choisi dans la gamme allant de $10^{-5}$ à $10^{-1}$, de préférence $10^{-3}$ à $10^{-2}$.

7. Utilisation selon l'une quelconque des revendications précédentes, **caractérisée en ce que** les sels de Mn(II) et de Zn(II) sont choisis parmi les chlorure, fluorure, iodure, sulfate, phosphate, nitrate, perchlorate, les sels d'acides carboxyliques et leurs mélanges.

8. Utilisation selon l'une quelconque des revendications précédentes, **caractérisée en ce que** le sel de Mn(II) et/ou de Zn(II) est le chlorure.

9. Utilisation selon la revendication 7, **caractérisée en ce que** les sels d'acides carboxyliques sont des sels d'acides carboxyliques hydroxylés.

10. Utilisation selon la revendication 7, **caractérisée en ce que** le sel d'acide carboxylique hydroxylé est le gluconate.

11. Utilisation selon l'une quelconque des revendications précédentes, **caractérisée en ce que** l'hydrogénocarbonate est choisi parmi l'hydrogénocarbonate de sodium, l'hydrogénocarbonate de potassium, l'hydrogénocarbonate de magnésium, l'hydrogénocarbonate de calcium et leurs mélanges.

12. Utilisation selon l'une quelconque des revendications précédentes, **caractérisée en ce que** le cycle aromatique comportant au moins deux groupes hydroxyles sur deux atomes de carbone consécutifs du précurseur de densification est un cycle benzénique ou un cycle aromatique condensé.

13. Utilisation selon l'une quelconque des revendications précédentes, **caractérisée en ce que** le précurseur de densification est un composé de formule (I) :

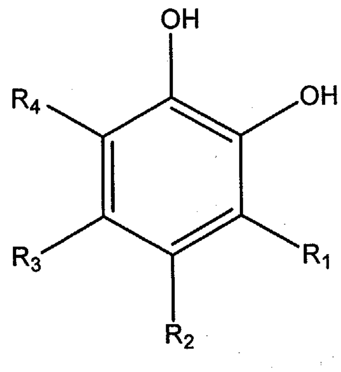

(I)

dans laquelle $R_1$ à $R_4$, identiques ou différents, représentent un atome d'hydrogène, un halogène, un radical hydroxyle, carboxyle, carboxylate d'alkyle, amino éventuellement substitué, alkyle linéaire ou ramifié éventuellement substitué, alcényle linéaire ou ramifié éventuellement substitué, cycloalkyle éventuellement substitué, alcoxy, alcoxyalkyle, alcoxyaryle, le groupe aryle pouvant être éventuellement substitué, aryle, aryle substitué, un radical hétérocyclique éventuellement substitué, un radical contenant éventuellement au moins un atome de silicium, ou deux des substituants $R_1$ à $R_4$ peuvent former conjointement un cycle saturé ou insaturé contenant éventuellement au moins un hétéroatome et éventuellement condensé avec au moins un cycle saturé ou insaturé contenant éventuellement au moins un hétéroatome.

**14.** Utilisation selon l'une quelconque des revendications précédentes, **caractérisée en ce que** le précurseur de densification est choisi parmi les flavonols, les flavonols, les anthocyanidines, les anthocyanines, les hydroxybenzoates, les flavones, les iridoïdes, les anthocyanines pouvant être éventuellement osylés et/ou sous forme d'oligomères, les hydroxystilbènes éventuellement osylés, la 3,4-dihydroxyphénylalanine et ses dérivés, la 2,3-dihydroxyphénylalanine et ses dérivés, la 4, 5-dihydroxyphénylalanine et ses dérivés, le 4,5-dihydroxyindole et ses dérivés, le 5,6-dihydroxyindole et ses dérivés, le 6,7-dihydroxyindole et ses dérivés, le 2,3-dihydroxyindole et ses dérivés, les dihydroxycinnamates, les hydroxycoumarines, les hydroxyisocoumarines, les hydroxycoumarones, les hydroxyisocoumarones, les hydroxychalcones, les hydroxychromones, les anthocyanes, les quinones, les hydroxyxantones, les 1,2-dihydroxybenzènes, les 1,2,4-trihydroxybenzènes, les 1,2,3-trihydroxybenzènes, le 2,4,5-trihydroxytoluène, le 5,6-dihydroxyindoline et leurs mélanges.

**15.** Utilisation selon l'une quelconque des revendications précédentes, **caractérisée en ce que** le précurseur de densification est choisi parmi les extraits de plantes, de fruits, d'agrumes, de légumes et leurs mélanges.

**16.** Utilisation selon l'une quelconque des revendications précédentes, **caractérisée en ce que** le précurseur de densification est choisi parmi les extraits de thé, de raisin, de pomme, de banane, de cacaotier, de sorgho, de pomme de terre et leurs mélanges.

**17.** Utilisation selon l'une quelconque des revendications précédentes, **caractérisée en ce que** le précurseur de densification est présent à raison d'au moins 0,001% du poids total de la composition.

**18.** Utilisation selon l'une quelconque des revendications précédentes, **caractérisée en ce que** le milieu physiologiquement acceptable est un milieu solubilisant du précurseur de densification, notamment à propriété bactériostatique.

**19.** Utilisation selon l'une quelconque des revendications précédentes, **caractérisée en ce que** le milieu physiologiquement acceptable comprend au moins un solvant du précurseur de densification.

**20.** Utilisation selon l'une quelconque des revendications précédentes, **caractérisée en ce que** le milieu physiologiquement acceptable comprend au moins un solvant du précurseur de densification choisi parmi l'eau, les alcools, les polyols, les éthers de polyols et leurs mélanges.

**21.** Utilisation selon l'une quelconque des revendications précédentes, **caractérisée en ce que** le milieu physiologiquement acceptable comprend, au moins un alcool choisi parmi les alcanols et les alcanediols.

**22.** Utilisation selon l'une quelconque des revendications précédentes, **caractérisée en ce que** le milieu physiologiquement acceptable comprend, **caractérisée en ce que** le solvant est un mélange eau/alcool.

**23.** Utilisation selon la revendication 22 ou 23, **caractérisée en ce que** l'alcool représente jusqu'à 80% en poids du mélange, de préférence 1 à 50% en poids et mieux de 5 à 20% en poids.

**24.** Utilisation selon l'une quelconque des revendications précédentes, **caractérisée en ce que** la composition est exempte de tout agent de chélation du sel de Mn(II) et/ou de Zn(II).

**25.** Utilisation selon l'une quelconque des revendications précédentes, **caractérisée en ce que** la composition est conditionnée sous forme d'un aérosol ou d'un système à pompe sans reprise d'air.

**26.** Utilisation selon l'une quelconque des revendications précédentes, **caractérisée en ce que** la composition se présente sous la forme de deux composants séparés, un premier composant comprenant le système catalytique, dissous dans un milieu physiologiquement acceptable, et un deuxième composant comprenant le précurseur de densification dissous dans un milieu physiologiquement acceptable.

**27.** Utilisation selon l'une quelconque des revendications précédentes, **caractérisée en ce que** la composition contient d'autres ingrédients choisis parmi les épaississants ou gélifiants de phase hydro-alcoolique ou de phase huileuse, les matières colorantes solubles dans le milieu de la composition, les charges, les pigments, les antioxydants, les conservateurs, les parfums, les électrolytes, les neutralisants, les agents bloqueurs d'U.V., les actifs cosmétiques et pharmaceutiques, leurs mélanges.

**28.** Procédé cosmétique de densification des cheveux humains ou de coiffage des cheveux, **caractérisé en ce qu'**il consiste à appliquer sur les cheveux et/ou le cuir chevelu, une composition cosmétique contenant, dans un milieu physiologiquement acceptable à application topique, une quantité efficace d'au moins un précurseur de densification, choisi parmi les composés comportant au moins un cycle aromatique ayant au moins deux groupes hydroxyles portés par deux atomes de carbone consécutifs du cycle aromatique, et une quantité efficace d'un système catalytique comprenant un premier constituant choisi parmi les sels et oxydes de Mn(II) et/ou de Zn(II) et leurs mélanges et un second constituant choisi parmi les hydrogénocarbonates alcalins, les hydrogénocarbonates alcalino-terreux et leurs mélanges, les proportions du premier et du second constituants étant telles que:

$$\frac{[Mn(II)]}{[HCO_3]} \leq 1 \text{ avec } [Mn(II)] \neq 0$$

$$[\frac{Zn(II)]}{[HC0_3]} \leq 1 \text{ avec } [Zn(II)] \neq 0$$

$$\frac{[Mn(II) + Zn(II)]}{[HCO_3]} \leq 1 \text{ avec } [Mn(II)] \text{ et } [Zn(II)] \neq 0$$

**29.** Composition de soin des fibres kératiniques humaines et/ou de la peau d'où émergent ces fibres, et plus spécialement une composition de soin des cheveux et/ou du cuir chevelu, contenant dans un milieu physiologiquement acceptable, a) une quantité efficace d'au moins un précurseur de densification choisi parmi les composés comportant au moins un cycle aromatique ayant au moins deux groupes hydroxyles (OH) portés par deux atomes de carbone consécutifs du cycle aromatique, b) une quantité efficace d'un système catalytique comprenant un premier constituant choisi parmi les sels et oxydes de Mn(II), et/ou Zn(II) et leurs mélanges et un second constituant choisi parmi les hydrogénocarbonates alcalins, les hydrogénocarbonates alcalino-terreux et leurs mélanges, et c) une quantité efficace d'au moins un actif à effet retardé augmentant la densité pilaire et/ou favorisant la repousse des fibres kératiniques notamment des cheveux et/ou limitant la chute de ces fibres et en particulier des cheveux, les proportions du premier constituant et du second constituants étant telles que :

$$\frac{[Mn(II)]}{[HCO_3]} \leq 1 \text{ avec } [Mn(II)] \neq 0$$

$$\left[\frac{Zn(II)]}{[HC0_3]} \leq 1 \text{ avec } [Zn(II)] \neq 0$$

$$\frac{[Mn(II) + Zn(II)]}{[HCO_3]} \leq 1 \text{ avec } [Mn(II)] \text{ et } [Zn(II)] \neq 0$$

où [Mn(II)], [Zn(II)] et [$HCO_3$] représentent respectivement les concentrations molaires en Mn(II), Zn(II) et $HCO_3$ dans la composition.

FIGURE

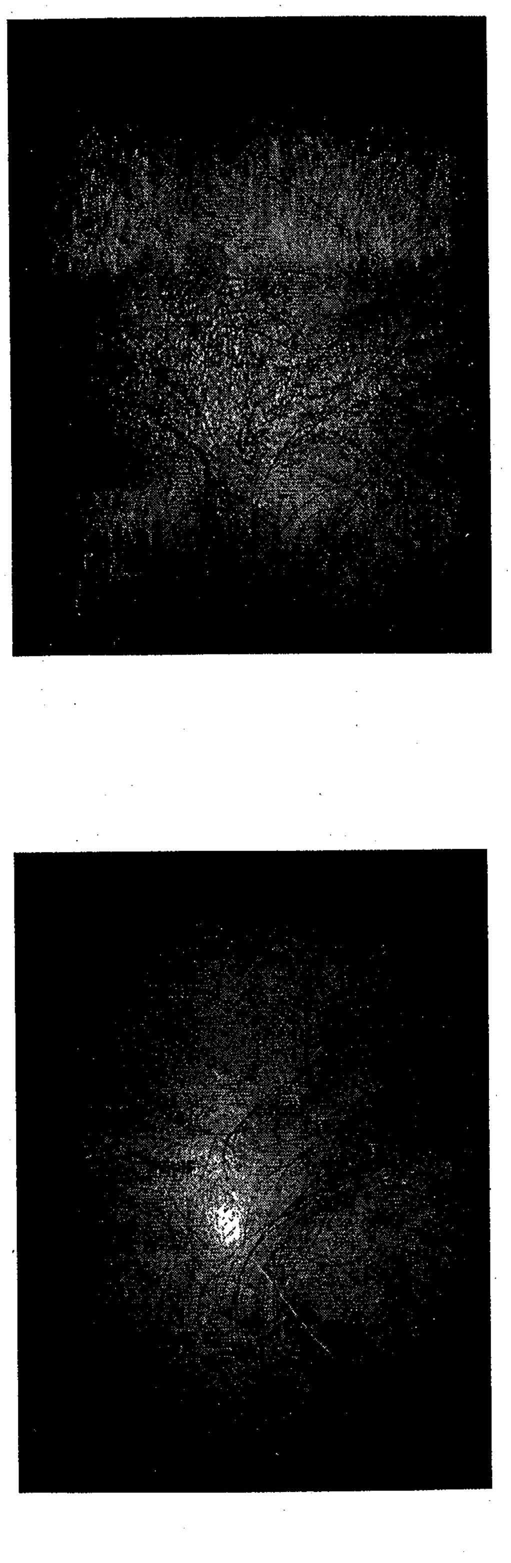

T=0

T=8 min